# EUROPEAN PATENT APPLICATION

(11) **EP 4 614 150 A1**
(43) Date of publication of application: **10.09.2025**
(21) Application number: 25162191.8
(22) Date of filing: 06.03.2025
(51) Int. Cl.: G01N 33/569

(54) **IMPROVED SYSTEMS AND PROCESSES TO SCREEN FOR SEVERE ACUTE RESPIRATORY SYNDROME CORONAVIRUS 2 (SARS-COV-2) OF 2019 (COVID-19)**

(30) Priority: 06.03.2024 US 202418597163
(71) Applicant: Battelle Memorial Institute, Columbus, OH 43201 (US)
(72) Inventor: GAINEY, Melicia, Columbus, 43201 (US); GARVER, Jennifer, Columbus, 43201 (US)
(74) Representative: Keltie LLP

(57) **Abstract**

The present disclosure provides systems and processes to screen for SARS-CoV-2. This disclosure teaches specific (and different) workable ranges for starting materials in a screening process for different SARS-CoV-2 variants (e.g., the Washington isolate, Alpha variant, Gamma P.1 variant, Beta variant, Iota variant, Delta variant, Omicron BA.1 variants, etc.). As shown herein, each variant has a different combination of starting materials and incubation periods, which further demonstrates the unpredictability of success that is associated with the disclosed systems and the disclosed processes. To be clear, the general ELISA process is well known by those having skill in the art. However, what is neither well known nor intuitive are the specific parameters associated with different process steps within ELISA. Those specific parameters are the subject of this disclosure.

## Description

### CROSS REFERENCE TO RELATED APPLICATIONS

This Application incorporates by reference the entireties of the disclosures from: U.S. Patent Application Serial Number 17/336,440, filed on June 2, 2021, having the title SYSTEMS AND PROCESSES TO SCREEN FOR SEVERE ACUTE RESPIRATORY SYNDROME CORONAVIRUS 2 (SARS-COV-2) OF 2019 (COVID-19); U.S. Provisional Patent Application Serial Number 63/041,551, filed June 19, 2020, having the title SYSTEMS AND PROCESSES TO SCREEN FOR SEVERE ACUTE RESPIRATORY SYNDROME CORONAVIRUS 2 (SARS-CoV-2) OF 2019 (COVID-19); U.S. Provisional Patent Application Serial Number 63/033,276, filed June 2, 2020, having the title SYSTEMS AND PROCESSES TO SCREEN FOR SEVERE ACUTE RESPIRATORY SYNDROME CORONAVIRUS 2 (SARS-CoV-2) OF 2019 (COVID-19) (hereafter, "Prior Applications").

### STATEMENT REGARDING FEDERALLY SPONSORED RESEARCH OR DEVELOPMENT

This invention was made with government support under HHSN272201800013I / 75N93020F00002 awarded by National Institute of Allergy and Infectious Diseases (NIAID), which is part of the National Institutes of Health (NIH), an agency of the United States Department of Health and Human Services (HHS). This invention was also made with government support under HHSO100201700011I / 75A50120F33012 (RTOR-AMT-1021), the United States Department of HHS. The government has certain rights in the invention.

### BACKGROUND

### FIELD OF THE DISCLOSURE

The present disclosure relates generally to Severe Acute Respiratory Syndrome (SARS) Coronavirus 2 (CoV-2) and, more particularly, to improved systems and processes to screen for SARS-CoV-2.

### DESCRIPTION OF RELATED ART

Screening for a virus, such as the Severe Acute Respiratory Syndrome Coronavirus-2 (SARS-CoV-2) of 2019 (COVID-19), can be done using Enzyme Linked Immunosorbent Assay (ELISA). ELISA involves at least one antibody with specificity for a particular antigen. As SARS-CoV-2 mutates and variants become more prevalent, previous approaches do not demonstrate usable titers when different starting materials are used. In other words, results are largely unpredictable for different starting materials, even when used in previously known assays, such as ELISA.

### SUMMARY

The present disclosure provides systems and processes to screen for SARS-CoV-2. This disclosure teaches specific (and different) workable ranges for starting materials in a screening process for different SARS-CoV-2 variants (e.g., the Washington strain (Wuhan), Alpha variant, Gamma P.1 variant, Beta variant, Iota variant, Washington isolate, Delta variant, Omicron BA.1 variant, Omicron BA.5 variant, and Omicron XBB.1.5 variant, etc.). As shown herein, each variant has a different combination of starting materials and incubation periods, which further demonstrates the unpredictability of success that is associated with the disclosed systems and the disclosed processes. To be clear, the general ELISA process is well known by those having skill in the art. However, what is neither well known nor intuitive are the specific parameters associated with different process steps of the assay. Those specific parameters are the subject of this disclosure.

Other systems, devices, methods, features, and advantages will be or become apparent to one with skill in the art upon examination of the following drawings and detailed description. It is intended that all such additional systems, methods, features, and advantages be included within this description, be within the scope of the present disclosure, and be protected by the accompanying claims.

### BRIEF DESCRIPTION OF THE DRAWINGS

Many aspects of the disclosure can be better understood with reference to the following drawings. The components in the drawings are not necessarily to scale, emphasis instead being placed upon clearly illustrating the principles of the present disclosure. Moreover, in the drawings, like reference numerals designate corresponding parts throughout the several views.

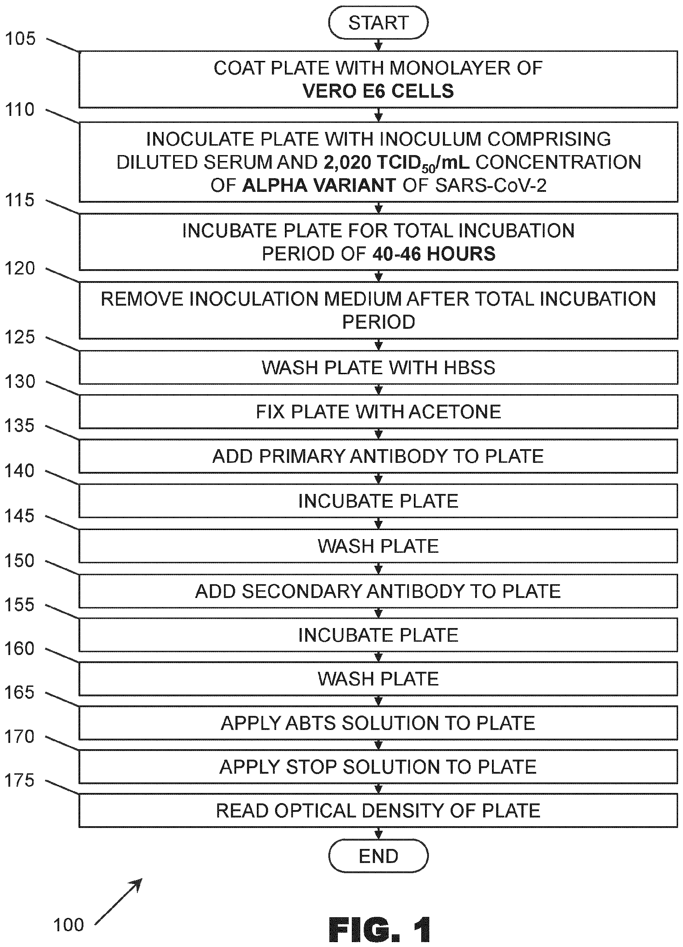

FIG. 1 is a flowchart showing an embodiment of a process for performing a microneutralization (MN) assay to quantify neutralizing antibodies for an Alpha variant of SARS-CoV-2 using Vero E6 cells (BEI Resources Catalog Number NR-596) at a target 2,020 fifty-percent-tissue-culture-infective-dose-assays-per-milliliter (TCID₅₀/mL) input concentration (equivalent to 202 TCID₅₀/well) and a total assay incubation period of between forty hours (40hrs) and forty-six hours (46hrs).

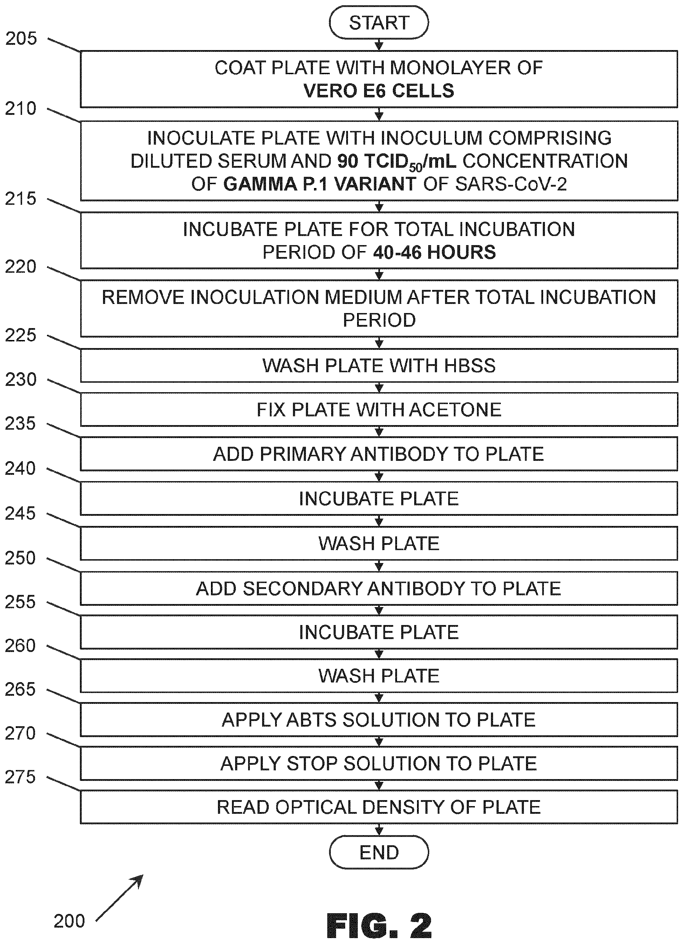

FIG. 2 is a flowchart showing an embodiment of a process for performing a MN assay to quantify neutralizing antibodies for a Gamma P.1 variant of SARS-CoV-2 using Vero E6 cells at a target 90 TCID₅₀/mL (equivalent to 9 TCID₅₀/well) input concentration and a total assay incubation period of between 40hrs and 46hrs.

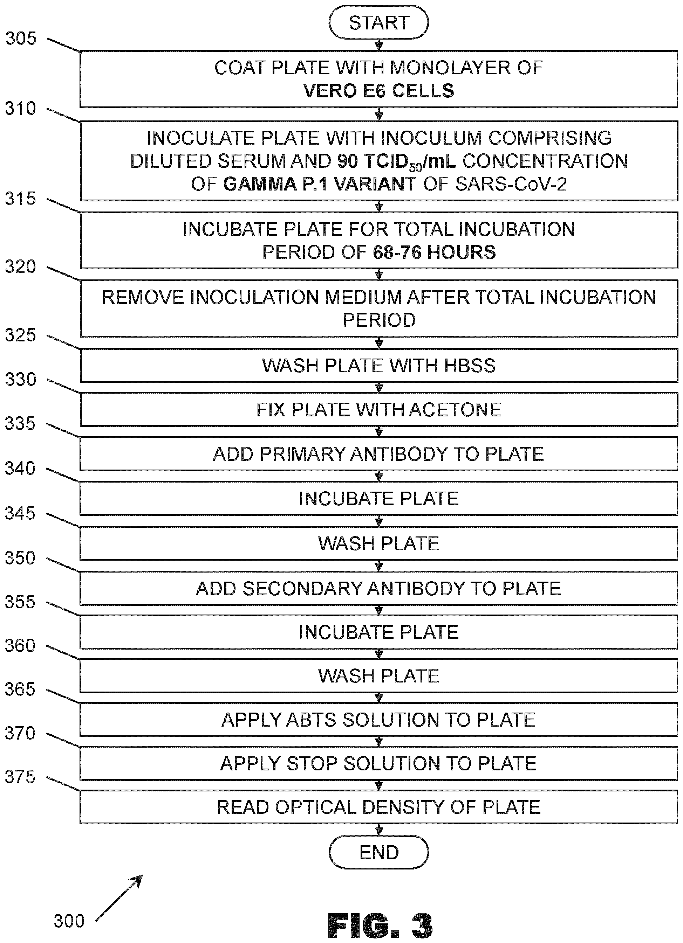

FIG. 3 is a flowchart showing an embodiment of a process for performing a MN assay to quantify neutralizing antibodies for a Gamma P.1 variant of SARS-CoV-2 using Vero E6 cells at a target 90 TCID₅₀/mL (equivalent to 9 TCID₅₀/well) input concentration and a total assay incubation period of between sixty-eight hours (68hrs) and seventy-six hours (76hrs).

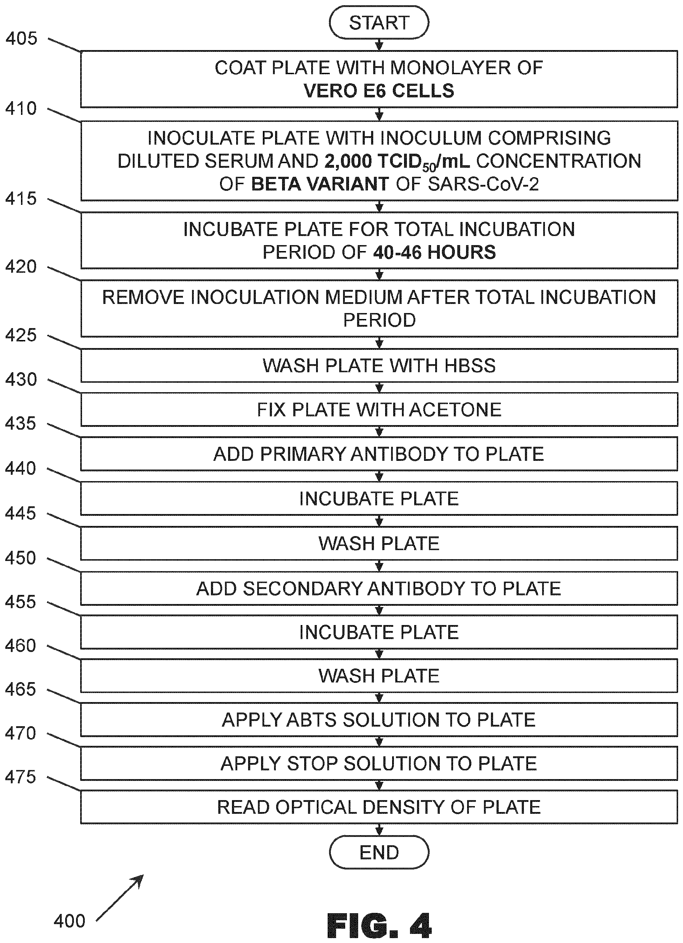

FIG. 4 is a flowchart showing an embodiment of a process for performing a MN assay to quantify neutralizing antibodies for a Beta variant of SARS-CoV-2 using Vero E6 cells at a target 2,000 TCID₅₀/mL (equivalent to 200 TCID₅₀/well) input concentration and a total assay incubation period of between 40hrs and 46hrs.

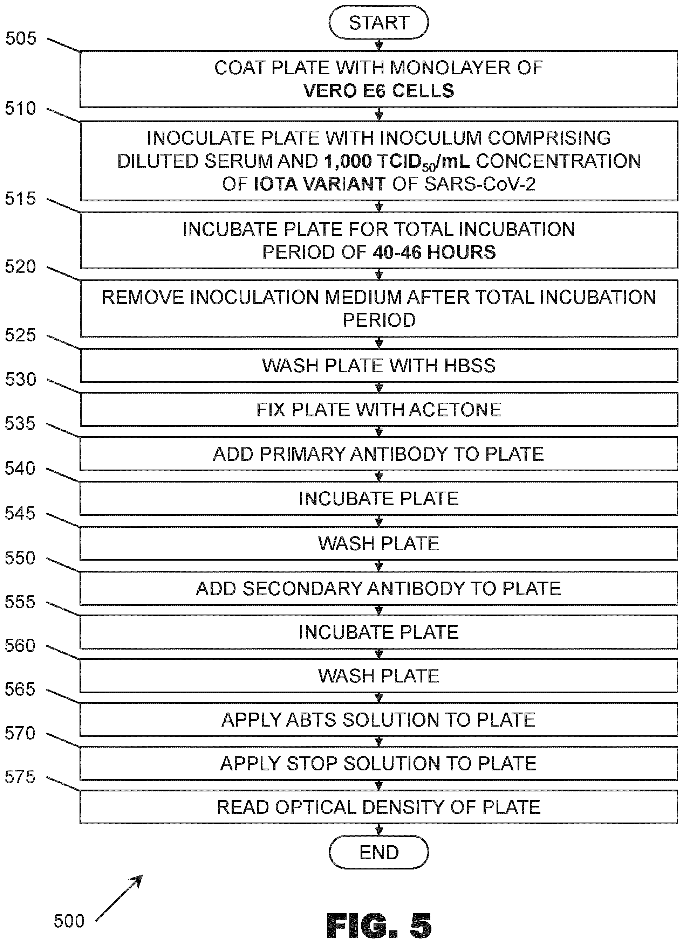

FIG. 5 is a flowchart showing an embodiment of a process for performing a MN assay to quantify neutralizing antibodies for an Iota variant of SARS-CoV-2 using Vero E6 cells at a target 1,000 TCID₅₀/mL (equivalent to 100 TCID₅₀/well) input concentration and a total assay incubation period of between 40hrs and 46hrs.

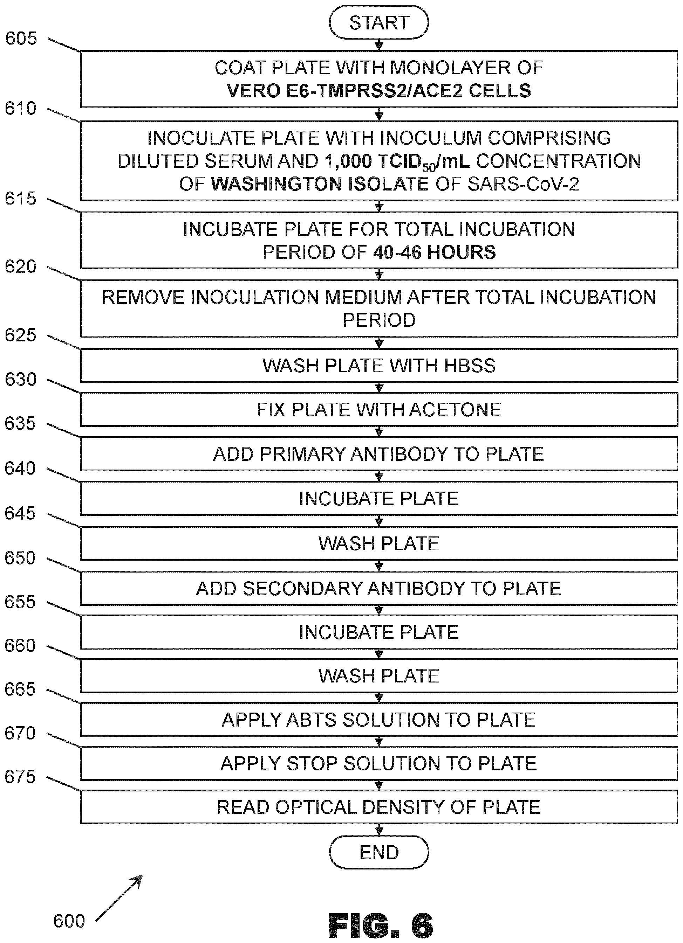

FIG. 6 is a flowchart showing an embodiment of a process for performing a MN assay to quantify neutralizing antibodies for a Washington isolate of SARS-CoV-2 using Vero E6 cells modified to express higher levels of transmembrane protease, serine 2 (TMPRSS2) and angiotensin-converting enzyme 2 (ACE2) (BEI Resources Catalog Number NR-54970) at a target 1,000 TCID₅₀/mL (equivalent to 100 TCID₅₀/well) input concentration and a total assay incubation period of between 40hrs and 46hrs.

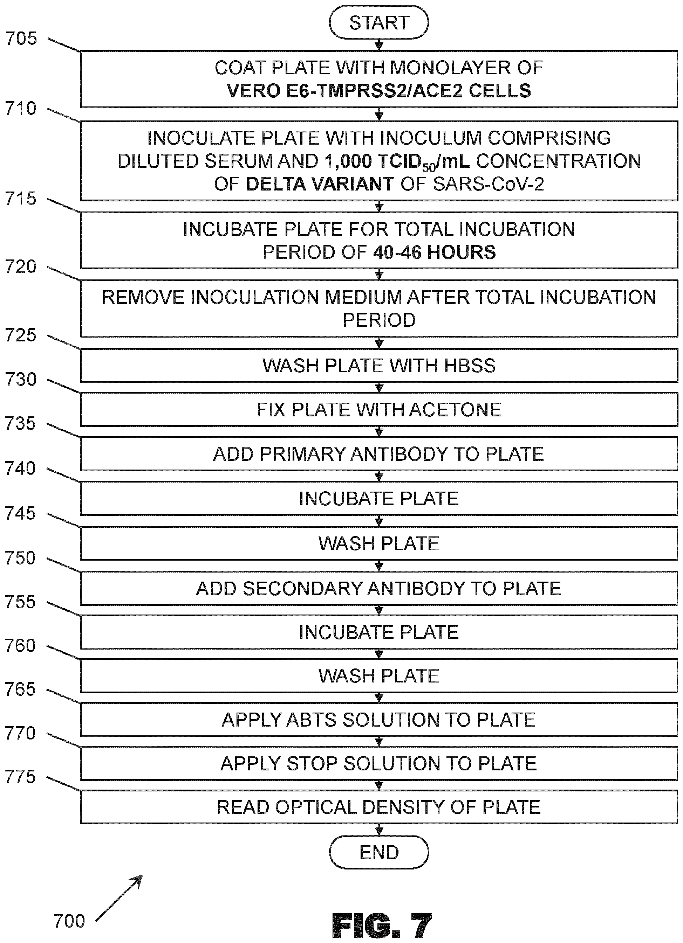

FIG. 7 is a flowchart showing an embodiment of a process for performing a MN assay to quantify neutralizing antibodies for a Delta variant of SARS-CoV-2 using Vero E6-TMPRSS2/ACE2 cells at a target 1,000 TCID₅₀/mL (equivalent to 100 TCID₅₀/well) input concentration and a total assay incubation period of between 40hrs and 46hrs.

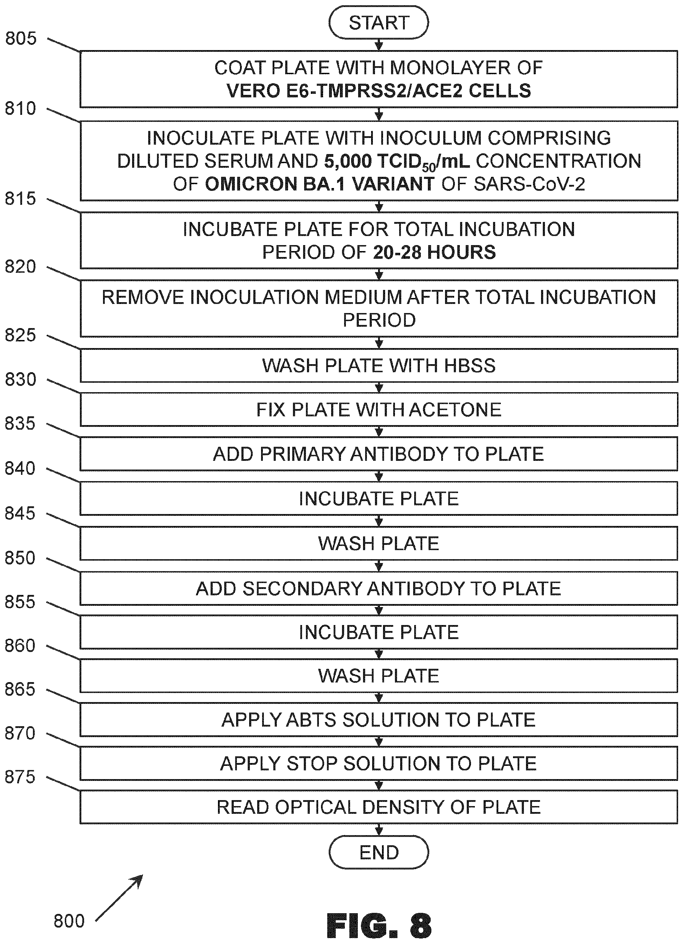

FIG. 8 is a flowchart showing an embodiment of a process for performing a MN assay to quantify neutralizing antibodies for an Omicron BA.1 variant of SARS-CoV-2 using Vero E6-TMPRSS2/ACE2 cells at a target 5,000 TCID₅₀/mL (equivalent to 500 TCID₅₀/well) input concentration and a total assay incubation period of between twenty hours (20hrs) and twenty-eight hours (28hrs).

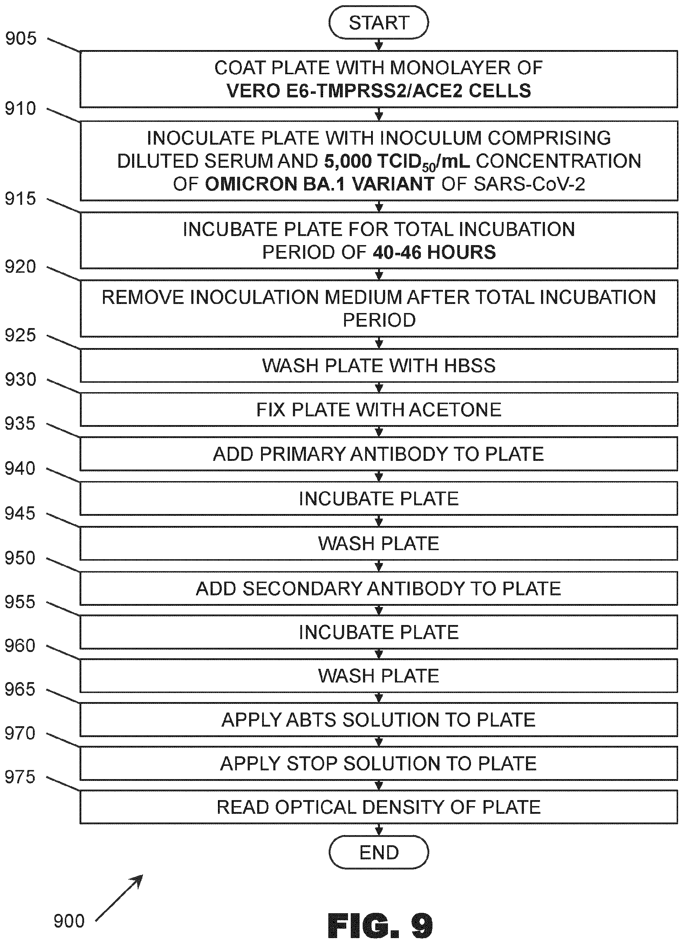

FIG. 9 is a flowchart showing an embodiment of a process for performing a MN assay to quantify neutralizing antibodies for an Omicron BA.1 variant of SARS-CoV-2 using Vero E6-TMPRSS2/ACE2 cells at a target 5,000 TCID₅₀/mL input concentration and a total assay incubation period of between 40hrs and 46hrs.

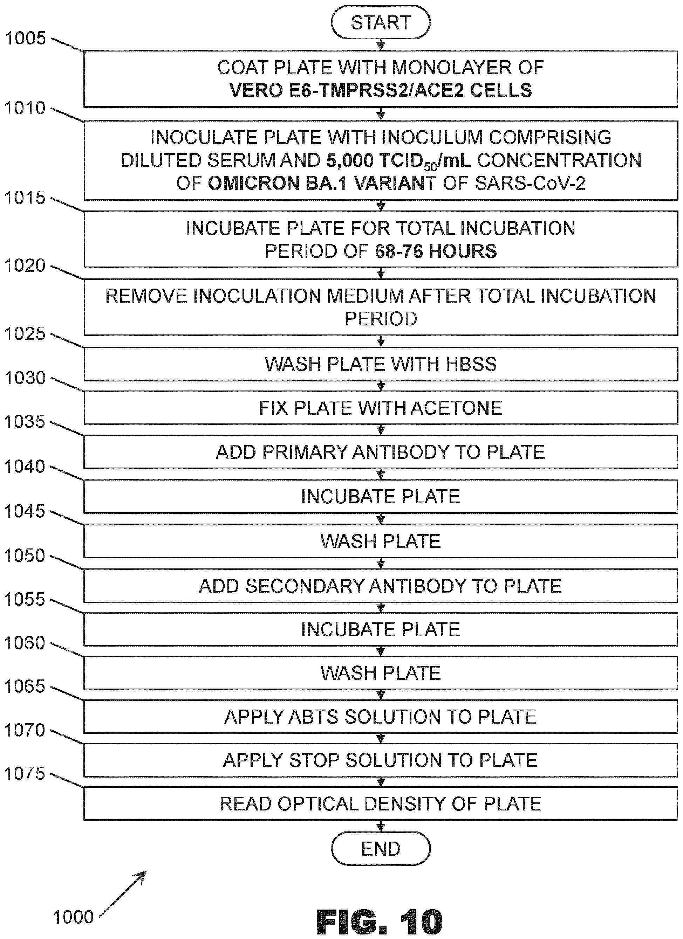

FIG. 10 is a flowchart showing an embodiment of a process for performing a MN assay to quantify neutralizing antibodies for an Omicron BA.1 variant of SARS-CoV-2 using Vero E6-TMPRSS2/ACE2 cells at a target 2,500 TCID₅₀/mL input concentration and a total assay incubation period of between 68hrs and 76hrs.

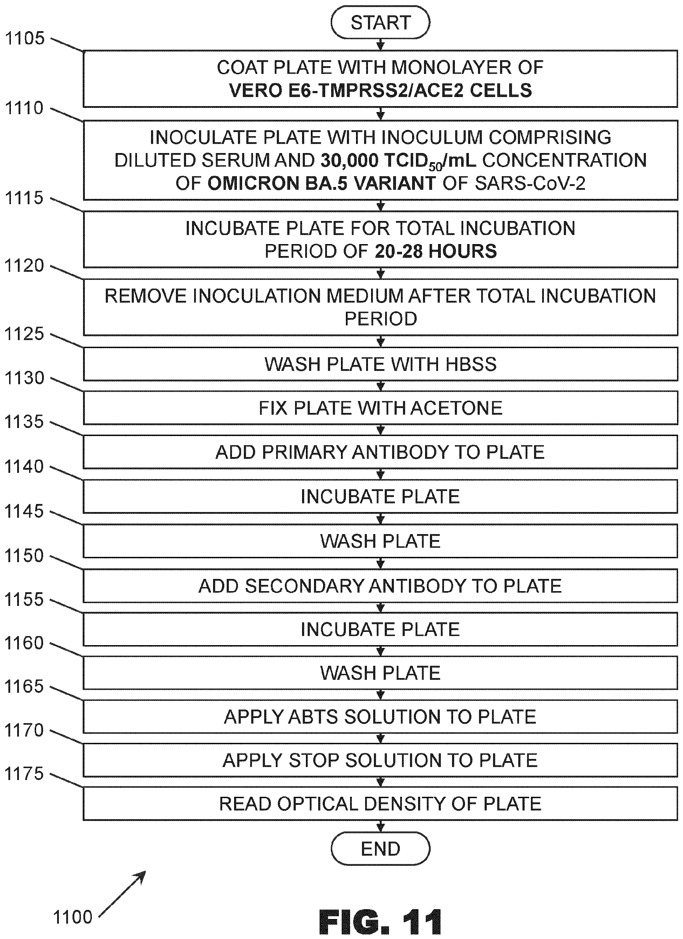

FIG. 11 is a flowchart showing an embodiment of a process for performing a MN assay to quantify neutralizing antibodies for an Omicron BA.5 variant of SARS-CoV-2 using Vero E6-TMPRSS2/ACE2 cells at a target 30,000 TCID₅₀/mL (equivalent to 3,000 TCID₅₀/well) input concentration and a total assay incubation period of between 20hrs and 28hrs.

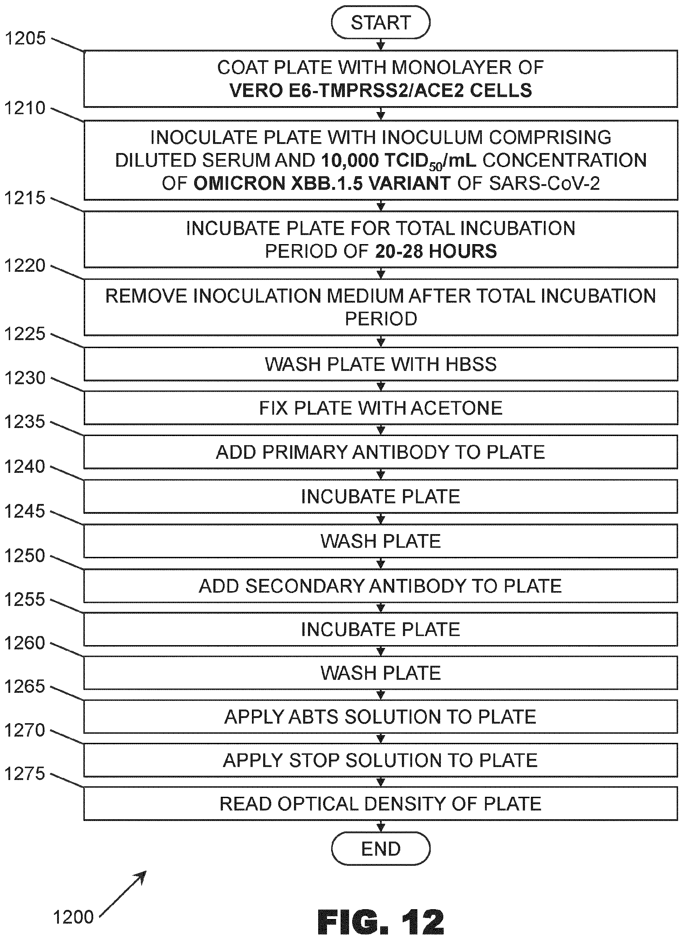

FIG. 12 is a flowchart showing an embodiment of a process for performing a MN assay to quantify neutralizing antibodies for an Omicron XBB.1.5 variant of SARS-CoV-2 using Vero E6-TMPRSS2/ACE2 cells at a target 10,000 TCID₅₀/mL (equivalent to 1,000 TCID₅₀/well) input concentration and a total assay incubation period of between 20hrs and 28hrs.

### DETAILED DESCRIPTION OF THE EMBODIMENTS

Screening for a virus, such as the Severe Acute Respiratory Syndrome Coronavirus-2 (SARS-CoV-2), can be done using Enzyme Linked Immunosorbent Assay (ELISA). ELISA involves at least one antibody with specificity for a particular antigen. In a global emergency, such as a pandemic (e.g., COVID-19 pandemic, etc.), there is a likelihood that a virus can mutate into different variants. Because the mutations to the virus are largely unpredictable, the efficacy of currently available treatments to the mutations is also largely unpredictable. As SARS-CoV-2 mutates and variants become more prevalent, previous approaches do not demonstrate usable titers when different starting materials are used. In other words, results are largely unpredictable for different starting materials, even when used in previously known assays, such as ELISA.

When devising potential screening processes for virus variants, those having skill in the art understand that there are numerous starting dilutions that can be used in a microneutralization (MN) assay. Because of the unpredictability of a reaction by a virus variant (or mutation or strain), there is no expectation of success that any particular starting dilution will be effective in a screening process for that particular virus strain (or variant or mutation). Similarly, although numerous cell lines can be used in the MN assay, there is (again) no expectation of success that any given cell line to be effective in the screening process of a particular virus variant (or mutation or strain). Likewise, while numerous virus titers can also be used in the MN assay, once again there is no expectation of success that any particular virus titer will be effective to screen for a particular virus variant (or mutation or strain). Compounding to the unpredictability is the sheer number of different combinations in which the numerous starting dilutions, numerous cell lines, and numerous virus titers can be combined, with each combination producing different outcomes. Additional exacerbating factors include different incubation times prior to fixation, which also affect effectiveness.

In other words, the sheer number of possible combinations, standing alone, makes it impractical to try every combination. Furthermore, because there is no expectation of success for any given combination, the process for determining which combination of dilutions, cell lines, and virus titers would be effective in a screening process is neither trivial nor intuitive. Even with Applicant's own work, which is disclosed herein, the required time to fully characterize starting materials took up to three (3) months per variant, which those having skill in the art will appreciate to be significant (in both time and resources). For some cases, the final timeline extended well beyond three (3) months and approached six (6) months.

Additionally, as the original virus mutates, and as the mutations themselves further mutate, into different strains (or variants) the potential screening processes for these newly emerging mutations requires additional time and resources, all of which are expended without any guarantee of success.

To address the emerging variants (or mutations or strains) of SARS-CoV-2, this disclosure teaches specific (and different) workable ranges for starting materials in a screening process for different SARS-CoV-2 variants (e.g., the Washington strain (Wuhan), Alpha variant, Gamma P.1 variant, Beta variant, Iota variant, Delta variant, and Omicron variants, etc.). As shown herein, each variant has a different combination of starting materials and incubation periods, which further demonstrates the unpredictability of success that is associated with the disclosed systems and the disclosed processes. To be clear, the general ELISA process is well known by those having skill in the art. However, what is neither well known nor intuitive are the specific parameters associated with different process steps within ELISA. Those specific parameters are the subject of this disclosure.

Having provided a broad technical solution to a technical problem, reference is now made in detail to the description of the embodiments as illustrated in the drawings. Specifically, FIGS. 1 through 12 are flowcharts showing various embodiments of processes for performing MN assays to quantify neutralizing antibodies in which different input viruses are used at varying virus concentrations using different starting materials (e.g. cell lines). Because results can vary widely with even marginal changes in input parameters, the primary steps of the processes are described separately for each embodiment, even though many of the steps may be the same from one embodiment to the next. For example, unless otherwise indicated, the *in situ* ELISA processes described in FIGS. 1 through 10 employed a standard 96-well configuration with the per-well volume being approximately 100 microliters (~100µL).

### 2,020 TCID₅₀/mL ALPHA VARIANT USING VERO E6 CELLS, INCUBATION 40-46 HOURS

With this in mind, FIG. 1 is a flowchart showing an embodiment of an *in situ* ELISA process 100 for performing a MN assay to quantify neutralizing antibodies for an Alpha variant of SARS-CoV-2 using Vero E6 cells (BEI Resources Catalog Number NR-596) at a target 2,020 fifty-percent-tissue-culture-infective-dose-assays-per-milliliter (TCID₅₀/mL) input concentration and a total assay incubation period of between forty hours (40hrs) and forty-six hours (46hrs).

In the process 100 of FIG. 1, a plate was coated 105 with a monolayer of Vero E6 cells. The plate was then inoculated 110 with 100µL/well inoculum, which included a pre-incubation with diluted immune serum and a target viral-infective dose of Severe Acute Respiratory Syndrome Coronavirus-2 (SARS-CoV-2). Specifically, after dilution in serum the viral-infective dose was 2,020 TCID₅₀/mL for a final Alpha variant input concentration of 202 TCID₅₀/well on the cell plate. The plate was then incubated 115 for a total incubation period of between 40hrs and 46hrs. Incubation 115 occurred at a temperature of ~37±2°C and a carbon dioxide (CO₂) content of ~5±2%CO₂.

After the total incubation period, the inoculation medium was removed 120 and the plate was washed 125 with Hanks Buffered Salt Solution (HBSS) at ~150µL/well. The HBSS wash was removed and the plate was fixed 130 with ~80% acetone. A primary antibody (such as purified mouse monoclonal anti-coronavirus (COVID-19, MERS, SARS-CoV) nucleoprotein antibody) was then added 135 to the plate and the plate was incubated 140 again at a temperature of ~37±2°C for approximately an hour (~60±5 minutes (min)). Specifically, ~297µL of blocking buffer and ~3µL of primary antibody was used for this particular incubation 140 period.

After incubation 140, the plate was washed 145 (preferably, at least three (3) times with ~300µL/well of wash buffer for each wash) and a secondary antibody (such as goat anti-mouse immunoglobulin G (IgG) conjugated with horseradish peroxidase) was added 150 to the plate. For this secondary antibody, the volume of the blocking buffer was ~11µL and the volume of the anti-mouse IgG conjugate was also ~11µL. Upon adding 150 the secondary antibody, the plate was again incubated 155 at a temperature of ~37±2°C for ~60±5min.

After the incubation 155, the plate was again washed 160 (preferably, at least three (3) times with ~300µL/well of wash buffer for each wash). Thereafter, a 2,2'-azino-bis(3-ethylbenzothiazoline-6-sulfonic acid) (ABTS) solution was applied 165 to the plate. After a period of ~30±5min, a stop solution was applied 170 to the plate.

The optical density (OD) of the plate was read 175 at 405 nanometers (nm) with a 490nm reference filter. An OD reading 175 over 0.7 was considered to be a reasonable threshold to show viral infection of the Alpha variant of SARS-CoV-2.

For clarity, in this embodiment, the workable range for screening the Alpha variant of SARS-CoV-2 was 2,020 TCID₅₀/mL input concentration after an equal dilution in serum with a total incubation period of 40hrs-46hrs. In other words, in order for the OD of 0.7 to properly indicate viral infection of the Alpha variant of SARS-CoV-2, the input concentration was 4,040 TCID₅₀/mL and was added to an equal volume of serum, followed by an initial ~1 hour neutralization incubation, after which a total concentration of 2,020 TCID₅₀/mL or 202 TCID₅₀/well virus inoculum containing serum was added to the cell plate with an incubation 115 period of 40hrs-46hrs. As shown below, the initial input concentrations and incubation periods differed based on the SARS-CoV-2 variant under study.

### 90 TCID₅₀/mL GAMMA P.1 VARIANT USING VERO E6 CELLS; INCUBATION 40-46 HOURS

FIG. 2 is a flowchart showing an embodiment of an *in situ* ELISA process 200 for performing a MN assay to quantify neutralizing antibodies for a Gamma P.1 variant of SARS-CoV-2 using Vero E6 cells at a target 90 TCID₅₀/mL input concentration and a total assay incubation period of between 40hrs and 46hrs. Although the total assay incubation in FIG. 2 (for the Gamma P.1 variant) is the same as the total assay incubation period in FIG. 1 (for the Alpha variant), it should be noted that target input concentration for the Alpha variant was more than twenty times (>20x) greater than the target input concentration for the Gamma P.1. variant.

Similar to FIG. 1, in the process 200 of FIG. 2, a plate was coated 205 with a monolayer of Vero E6 cells. The plate was then inoculated 210 with 100µL/well inoculum, which included a pre-incubation with diluted immune serum and a target viral-infective dose of SARS-CoV-2. Specifically, after dilution in serum the viral-infective dose was 90 TCID₅₀/mL for a final input concentration of the Gamma P.1 variant of SARS-CoV-2 of 9 TCID₅₀/well (again, much less than the concentration of the Alpha variant of FIG. 1). The plate was then incubated 215 for a total incubation period of 40hrs-46hrs at a temperature of ~37±2°C and ~5±2%CO₂.

Thereafter, the inoculation medium was removed 220 and the plate was washed 225 with HBSS at ~150µL/well. The HBSS wash was then removed and the plate was fixed 230 with ~80% acetone. A primary antibody was then added 235 to the plate and the plate was incubated 240 again at a temperature of ~37±2°C for ~60±5min. The plate was then washed 245 again and a secondary antibody was added 250 to the plate. Upon adding 250 the secondary antibody, the plate was again incubated 255 at a temperature of ~37±2°C for ~60±5min. After this incubation 255, the plate was again washed 260, ABTS solution was applied 265 to the plate. Shortly thereafter, a stop solution was applied 270 to the plate and the OD of the plate was read 175, with an OD > 0.7 being considered as the threshold to show viral infection of the Gamma P.1 variant of SARS-CoV-2.

In other words, the workable range for screening the Gamma P.1 variant of SARS-CoV-2 was 90 TCID₅₀/mL input concentration (much lower than the 2,020 TCID₅₀/mL concentration for the Alpha variant).

### 90 TCID₅₀/mL GAMMA P.1 VARIANT USING VERO E6 CELLS; INCUBATION 68-76 HOURS

FIG. 3 also shows an *in situ* ELISA process 300 for the Gamma P.1 variant of SARS-CoV-2. Unlike FIG. 2 (which showed a total initial incubation period of 40hrs-46hrs), the process 300 of FIG. 3 incubates 315 the plate for a total incubation period of sixty-eight hours (68hrs) to seventy-six (76) hours. Because this incubation 315 step is the only significant difference between FIG. 2 and FIG. 3, the remaining steps of the process 300 of FIG. 3 are only described briefly.

In the process 300 of FIG. 3, the plate was coated 305 with a monolayer of Vero E6 cells, inoculated 310 with an inoculum (diluted serum and 90 TCID₅₀/mL concentration of the Gamma P.1 variant), and incubated 315 for a total incubation period of 68hrs-76hrs. Thereafter, the inoculation medium was removed 320, the plate washed 325 with HBSS, and fixed 330 with acetone. A primary antibody was added 335 to the plate and the plate was thereafter incubated 340 and washed 345. After washing 345, a secondary antibody was added 350 and the plate was again incubated 355 and washed 360. ABTS solution was applied 365 to the plate and, shortly thereafter, a stop solution was applied 370. The OD of the plate was read 375, with an OD > 0.7 being considered as the threshold to show viral infection of the Gamma P.1 variant of SARS-CoV-2.

Although both 40hrs-46hrs of incubation (FIG. 2) and 68hrs-76hrs of incubation (FIG. 3) were studied, the 40hrs-46hrs of incubation (FIG. 2) is the preferred incubation period for the Gamma P.1 variant of SARS-CoV-2.

### 2,000 TCID₅₀/mL BETA VARIANT USING VERO E6 CELLS; INCUBATION 40-46 HOURS

Next, FIG. 4 is a flowchart showing an embodiment of an *in situ* ELISA process 400 for performing a MN assay to quantify neutralizing antibodies for a Beta variant of SARS-CoV-2 using Vero E6 cells at a target 2,000 TCID₅₀/mL input concentration and a total assay incubation period of between 40hrs and 46hrs. Comparatively, the input concentration of the Beta variant (FIG. 4) is closer to the input concentration of the Alpha variant (FIG. 1 (2,020 TCID₅₀/mL Alpha variant)) than it is to the input concentration of the Gamma P.1 variant (FIGS. 2 and 3 (90 TCID₅₀/mL Gamma P.1 variant)).

In the process 400 of FIG. 4, a plate was coated 405 with a monolayer of Vero E6 cells. The plate was then inoculated 410 with 100µL/well inoculum, which included a pre-incubation with diluted immune serum and a target viral-infective dose of SARS-CoV-2. Specifically, after dilution in serum the viral-infective dose was 2,000 TCID₅₀/mL for a final Beta variant input concentration of 200 TCID₅₀/well on the cell plate. The plate was then incubated 415 for a total incubation period of 40hrs-46hrs at a temperature of ~37±2°C and ~5±2%CO₂.

Thereafter, the inoculation medium was removed 420 and the plate was washed 425 with HBSS. The HBSS wash was then removed and the plate was fixed 430 with acetone. A primary antibody was then added 435 to the plate and the plate was incubated 440 again. Thereafter, the plate was washed 445 again and a secondary antibody was added 450 to the plate. Upon adding 450 the secondary antibody, the plate was again incubated 455 and, after this incubation 455, the plate was again washed 460. ABTS solution was applied 465 to the plate and, shortly thereafter, a stop solution was applied 470 to the plate. The OD of the plate was read 475, with OD > 0.7 being considered as the threshold to show viral infection of the Beta variant of SARS-CoV-2.

For clarity, the workable range for screening the Beta variant of SARS-CoV-2 was 2,000 TCID₅₀/mL input concentration (closer to the Alpha variant and considerably different from the Gamma P.1 variant).

### 1,000 TCID₅₀/mL IOTA VARIANT USING VERO E6 CELLS, INCUBATION 40-46 HOURS

Continuing, FIG. 5 is a flowchart showing an embodiment of a process for performing a MN assay to quantify neutralizing antibodies for an Iota variant of SARS-CoV-2 using Vero E6 cells at a target 1,000 TCID₅₀/mL input concentration and a total assay incubation period of between 40hrs and 46hrs. The 1,000 TCID₅₀/mL input concentration for the Iota variant resides at approximately the midpoint between the Gamma P.1 variant (FIGS. 2 and 3) and the Alpha or Beta variants (FIGS. 1 and 4). As one having skill in the art can appreciate, the input concentration for the Iota variant (FIG. 5) is remarkably different than the input concentrations for the Alpha variant (FIG. 1), Beta variant (FIG. 4), or the Gamma P.1 variant (FIGS. 2 and 3).

With this in mind, the process 500 of FIG. 5 comprises coating 505 a plate with a monolayer of Vero E6 cells and inoculating 510 the coated plate with 100µL/well inoculum (which included a pre-incubation of diluted immune serum and a target viral-infective dose of SARS-CoV-2. Specifically, after dilution in serum the viral-infective dose was 1,000 TCID₅₀/mL for a final Iota variant input concentration 100 TCID₅₀/well on the cell plate). The plate was then incubated 515 for a total incubation period of 40hrs-46hrs at a temperature of ~37±2°C and ~5±2%CO₂. The inoculation medium was removed 520 and the plate was washed 525 with HBSS. The HBSS wash was then removed and the plate was fixed 530 with acetone. A primary antibody was then added 535 to the plate and the plate was again incubated 540. Thereafter, the plate was washed 545 and a secondary antibody was added 550 to the plate. Upon adding 550 the secondary antibody, the plate was again incubated 555 and, after this incubation 555, the plate was again washed 560. ABTS solution was applied 565 to the plate and, shortly thereafter, a stop solution was applied 570 to the plate. The OD of the plate was read 575, with OD > 0.7 being considered as the threshold to show viral infection of the Iota variant of SARS-CoV-2.

The workable range for screening the Iota variant of SARS-CoV-2 was 1,000 TCID₅₀/mL input concentration, with an initial total incubation period for the assay being 40hrs-46hrs.

### 1,000 TCID₅₀/mL WASHINGTON ISOLATE USING VERO E6-TMPRSS2/ACE2 CELLS, INCUBATION 40-46 HOURS

FIGS. 1 through 5 showed processes in which the plates were coated with Vero E6 cells. FIGS. 6 through 10 show processes in which plates are coated Vero E6 cells modified to express higher levels of transmembrane protease, serine 2 (TMPRSS2) and angiotensin-converting enzyme 2 (ACE2) (BEI Resources Catalog Number NR-54970) (Vero E6-TMPRSS2/ACE2).

With this in mind, FIG. 6 is a flowchart showing an embodiment of a process for performing a MN assay to quantify neutralizing antibodies for a Washington isolate of SARS-CoV-2 using Vero E6-TMPRSS2/ACE2 at a target 1,000 TCID₅₀/mL input concentration and a total assay incubation period of between 40hrs and 46hrs.

The process 600 of FIG. 6 comprises coating 605 a plate with a monolayer of Vero E6-TMPRSS2/ACE2 cells and inoculating 610 the coated plate with 100µL/well inoculum (which included a pre-incubation of diluted immune serum and a target viral-infective dose of SARS-CoV-2. Specifically, after dilution in serum the viral-infective dose was 1,000 TCID₅₀/mL for a final Washington isolate input concentration of 100 TCID₅₀/well on the cell plate). The plate was then incubated 615 for a total incubation period of 40hrs-46hrs at a temperature of ~37±2°C and ~5±2%CO₂. The inoculation medium was removed 620 and the plate was washed 625 with HBSS. The HBSS wash was then removed and the plate was fixed 630 with acetone. A primary antibody was then added 635 to the plate and the plate was again incubated 640. Thereafter, the plate was washed 645 and a secondary antibody was added 650 to the plate. Upon adding 650 the secondary antibody, the plate was again incubated 655 and, after this incubation 655, the plate was again washed 660. As before, ABTS solution was applied 665, followed by a stop solution being applied 670 to the plate. The OD of the plate was read 675, with OD > 0.7 being considered as the threshold to show viral infection of the Washington isolate of SARS-CoV-2.

The suitable range for screening the Washington isolate of SARS-CoV-2 was 1,000 TCID₅₀/mL input concentration, with an initial total incubation period for the assay being 40hrs-46hrs. Although these parameters for the Washington isolate (FIG. 6) may appear to be similar to the parameters for the Iota variant (FIG. 5), the primary difference is that the Iota variant (FIG. 5) used Vero E6 cells, while the Washington isolate (FIG. 6) used the Vero E6-TMPRSS2/ACE2 cells.

### 1,000 TCID₅₀/mL DELTA VARIANT USING VERO E6-TMPRSS2/ACE2 CELLS, INCUBATION 40-46 HOURS

FIG. 7 is a flowchart showing an embodiment of a process for performing a MN assay to quantify neutralizing antibodies for a Delta variant of SARS-CoV-2 using Vero E6-TMPRSS2/ACE2 cells at a target 1,000 TCID₅₀/mL input concentration and a total assay incubation period of between 40hrs and 46hrs. The suitable range for the Delta variant (FIG. 7) is substantially similar to the suitable range for the Washington isolate (FIG. 6). Thus, only a truncated discussion of the process 700 for the Delta variant is provided.

The process 700 of FIG. 7 comprises coating 705 a plate with a monolayer of Vero E6-TMPRSS2/ACE2 cells and inoculating 710 the coated plate with 100µL/well inoculum (which included a pre-incubation of diluted immune serum and a target viral-infective dose of SARS-CoV-2. Specifically, after dilution in serum the viral-infective dose was 1,000 TCID₅₀/mL for a final Delta variant input concentration of 100 TCID₅₀/well on the cell plate). The plate was then incubated 715 for a total incubation period of 40hrs-46hrs at a temperature of ~37±2°C and ~5±2%CO₂. The inoculation medium was removed 720 from the plate, the plate was washed 725 with HBSS, and the plate was then fixed 730 with acetone. A primary antibody was then added 735 to the plate and the plate was again incubated 740. Thereafter, the plate was again washed 745 and a secondary antibody was added 750 to the plate. Upon adding 750 the secondary antibody, the plate was again incubated 755 and, after this incubation 755, the again washed 760. As before, ABTS solution was applied 665, followed by a stop solution being applied 770. Thereafter, OD of the plate was read 775, with OD > 0.7 being considered as the threshold to show viral infection of the Delta variant of SARS-CoV-2.

Briefly, the suitable range for screening the Delta variant of SARS-CoV-2 was 1,000 TCID₅₀/mL input concentration, incubated for 40hrs-46hrs.

### 5,000 TCID₅₀/mL OMICRON BA.1 VARIANT USING VERO E6-TMPRSS2/ACE2 CELLS, INCUBATION 20-28 HOURS

FIGS. 8, 9, 10 show processes 800, 900, 1000 for the Omicron BA.1 variant of COVID-19 with assay incubation periods of 20hrs-28hrs, 40hrs-46hrs, and 68hrs-76hrs, respectively.

FIG. 8 is a flowchart showing an embodiment of a process for performing a MN assay to quantify neutralizing antibodies for an Omicron BA.1 variant of SARS-CoV-2 using Vero E6-TMPRSS2/ACE2 cells at a target 5,000 TCID₅₀/mL input concentration and a total assay incubation period of between twenty hours (20hrs) and twenty-eight hours (28hrs).

Notably, the input concentration of 5,000 TCID₅₀/mL for the Omicron BA.1 variant (FIG. 8) is: (a) more-than double (>2x) the input concentrations for the Alpha variant (FIG. 1) and the Beta variant (FIG. 4); (b) five times (5x) greater than the input concentrations for the Iota variant (FIG. 5), the Washington isolate (FIG. 6), and the Delta variant (FIG. 7), and (c) more than fifty-five times (>55x) greater than the input concentration for the Gamma P.1 variant (FIGS. 2 and 3).

With this in mind, the process 800 of FIG. 8 comprises coating 805 a plate with a monolayer of Vero E6-TMPRSS2/ACE2 cells and inoculating 810 the coated plate with 100µL/well inoculum (which included a pre-incubation of diluted immune serum and a target viral-infective dose of SARS-CoV-2. Specifically, after dilution in serum the viral-infective dose was 5,000 TCID₅₀/mL for a final Omicron BA.1 variant input concentration 500 TCID₅₀/well on the cell plate). The plate was then incubated 815 for a total incubation period of 20hrs-28hrs at a temperature of ~37±2°C and ~5±2%CO₂. The inoculation medium was removed 820 from the plate, the plate washed 825 and fixed 830 with acetone, and a primary antibody added 835 to the plate. The plate was again incubated 840 and washed 845, followed by a secondary antibody being added 850 to the plate. Upon adding 850 the secondary antibody, the plate was again incubated 855 and washed 860. ABTS solution was applied 865, followed by a stop solution being applied 870. Similar to FIGS. 1 through 7, the OD of the plate was read 875, with OD > 0.7 being considered as the threshold to show viral infection of the Omicron BA.1 variant of SARS-CoV-2.

The suitable range for screening the Omicron BA.1 variant of COVID-19 using Vero E6-TMPRSS2/ACE2 was 5,000 TCID₅₀/mL for the input concentration and an initial total incubation period of 20hrs-28hrs.

### 5,000 TCID₅₀/mL OMICRON BA.1 VARIANT USING VERO E6-TMPRSS2/ACE2 CELLS; INCUBATION 40-46 HOURS

FIG. 9 also shows an *in situ* ELISA process 900 for the Omicron BA.1 variant of SARS-CoV-2. Unlike FIG. 8 (which showed a total initial incubation period of 20hrs-28hrs), the process 900 of FIG. 9 incubates 915 the plate for a total initial incubation period of 40hrs-46hrs. Because this incubation 915 step is the only significant difference between FIG. 8 and FIG. 9, the remaining steps of the process 900 of FIG. 9 are only described briefly.

In the process 900 of FIG. 9, the plate was coated 905 with a monolayer of Vero E6-TMPRSS2/ACE2 cells, inoculated 910 with 100µL/well inoculum (which included a pre-incubation of diluted immune serum and a target viral-infective dose of SARS-CoV-2. Specifically, after dilution in serum the viral-infective dose was 5,000 TCID₅₀/mL for a final Omicron BA.1 variant input concentration of 500 TCID₅₀/well on the cell plate), and incubated 915 for a total incubation period of 40hrs-46hrs. Thereafter, the inoculation medium was removed 920, the plate washed 925 with HBSS, and fixed 930 with acetone. A primary antibody was added 935 to the plate and the plate was thereafter incubated 940 and then washed 945. After washing 945, a secondary antibody was added 950 and the plate was again incubated 955 and thereafter washed 960. ABTS solution was applied 965 to the plate and, shortly thereafter, a stop solution was applied 970. Again, the OD of the plate was read 975, with OD > 0.7 being considered as the threshold to show viral infection of the Omicron BA.1 variant of SARS-CoV-2.

### 5,000 TCID₅₀/mL OMICRON BA.1 VARIANT USING VERO E6-TMPRSS2/ACE2 CELLS; INCUBATION 68-76 HOURS

FIG. 10 also shows an *in situ* ELISA process 1000 for the Omicron BA.1 variant of SARS-CoV-2. Unlike FIGS. 8 or 9 (which showed a total initial incubation period of 20hrs-28hrs and 40hrs-46hrs, respectively), the process 1000 of FIG. 10 incubates 1015 the plate for a total initial incubation period of 68hrs-76hrs. Because this incubation 1015 step is the only significant difference between FIG. 10 and FIGS. 8 and 9, the remaining steps of the process 1000 of FIG. 10 are only described briefly.

In the process 1000 of FIG. 10, the plate was coated 1005 with a monolayer of Vero E6-TMPRSS2/ACE2 cells, inoculated 1010 with 100µL/well inoculum (which included a pre-incubation of diluted immune serum and a target viral-infective dose of SARS-CoV-2. Specifically, after dilution in serum the viral-infective dose was 5,000 TCID₅₀/mL for a final Omicron BA.1 variant input concentration of 500 TCID₅₀/well on the cell plate), and incubated 1015 for a total incubation period of 68hrs-76hrs. Thereafter, the inoculation medium was removed 1020, the plate washed 1025 with HBSS, and fixed 1030 with acetone. A primary antibody was added 1035 to the plate and the plate was thereafter incubated 1040 and then washed 1045. After washing 1045, a secondary antibody was added 1050 and the plate was again incubated 1055 and thereafter washed 1060. ABTS solution was applied 1065 to the plate and, shortly thereafter, a stop solution was applied 1070. Once again, the OD of the plate was read 1075, with OD > 0.7 being considered as the threshold to show viral infection of the Omicron BA.1 variant of SARS-CoV-2.

Although 20hrs-28hrs (FIG. 8), 40hrs-46hrs (FIG. 9), and 68hrs-76hrs (FIG. 10) were studied, the 20hrs-28hrs of incubation (FIG. 8) is preferable as the incubation period for the Omicron BA.1 variant of COVID-19.

### 30,000 TCID₅₀/mL OMICRON BA.5 VARIANT USING VERO E6-TMPRSS2/ACE2 CELLS; INCUBATION 20-28 HOURS

FIG. 11 shows an *in situ* ELISA process 1100 for the Omicron BA.5 variant of SARS-CoV-2. In the process 1100 of FIG. 11, the plate was coated 1105 with a monolayer of Vero E6-TMPRSS2/ACE2 cells, inoculated 1110 with 100µL/well inoculum (which included a pre-incubation of diluted immune serum and a target viral-infective dose of SARS-CoV-2. Specifically, after dilution in serum the viral-infective dose was 30,000 TCID₅₀/mL for a final Omicron BA.5 variant input concentration of 3,000 TCID₅₀/well on the cell plate), and incubated 1115 for a total incubation period of 20hrs-28hrs. Thereafter, the inoculation medium was removed 1120, the plate washed 1125 with HBSS, and fixed 1130 with acetone. A primary antibody was added 1135 to the plate and the plate was thereafter incubated 1140 and then washed 1145. After washing 1145, a secondary antibody was added 1150 and the plate was again incubated 1155 and thereafter washed 1160. ABTS solution was applied 1165 to the plate and, shortly thereafter, a stop solution was applied 1170. Again, the OD of the plate was read 1175, with OD > 0.7 being considered as the threshold to show viral infection of the Omicron BA.5 variant of 11SARS-CoV-2.

It should be noted that FIG. 11 in conjunction with FIGS. 2 and 3 demonstrate the large variability in ranges for starting material. As shown by the numerical values, there is a greater-than three-hundred-and-thirty-three times (>333x) variability between the Gamma P.1 variant (FIGS. 2 and 3 at 90 TCID₅₀/mL) and the Omicron B.5 variant (FIG. 11 at 30,000 TCID₅₀/mL). To determine the precise numerical value for a usable titer is not a simple matter, as it is impractical to try every possible combination of starting values.

### 10,000 TCID₅₀/mL OMICRON XBB.1.5 VARIANT USING VERO E6-TMPRSS2/ACE2 CELLS; INCUBATION 20-28 HOURS

FIG. 12 also shows an *in situ* ELISA process 1200 for the Omicron BA.1 variant of SARS-CoV-2.

In the process 1200 of FIG. 12, the plate was coated 1205 with a monolayer of Vero E6-TMPRSS2/ACE2 cells, inoculated 1210 with 100µL/well inoculum (which included a pre-incubation of diluted immune serum and a target viral-infective dose of SARS-CoV-2. Specifically, after dilution in serum the viral-infective dose was 10,000 TCID₅₀/mL for a final Omicron XBB.1.5 variant input concentration of 1,000 TCID₅₀/well on the cell plate), and incubated 1215 for a total incubation period of 20hrs-28hrs. Thereafter, the inoculation medium was removed 1220, the plate washed 1225 with HBSS, and fixed 1230 with acetone. A primary antibody was added 1235 to the plate and the plate was thereafter incubated 1240 and then washed 1245. After washing 1245, a secondary antibody was added 1250 and the plate was again incubated 1255 and thereafter washed 1260. ABTS solution was applied 1265 to the plate and, shortly thereafter, a stop solution was applied 1270. Once again, the OD of the plate was read 1275, with OD > 0.7 being considered as the threshold to show viral infection of the Omicron XBB.1.5 variant of SARS-CoV-2.

### VARIABILITY AS SHOWN IN FIGS. 1 THROUGH 12

As shown in FIGS. 1 through 12, different mutations (e.g., Alpha, Beta, Gamma P.1, Delta, Iota, Omicron BA.1, Omicron BA.5, Omicron XBB.1.5, Washington isolate, etc.) for SARS-CoV-2 exhibit widely varying input concentrations for detectability at OD > 0.7. For example, there is a greater-than three-hundred-and-thirty-three times (>333x) variability between the Gamma P.1 variant (FIGS. 2 and 3 at 90 TCID₅₀/mL) and the Omicron BA.5 variant (FIG. 11 at 30,000 TCID₅₀/mL). Thus, one cannot simply use the same titers across all variants. In other words, the usable titers differ widely when different starting materials are used. This large degree of unpredictability means that no particular starting dilution can be expected to produce successful results and the effectiveness in screening differs from one particular virus strain (or variant or mutation) to another. Similarly, because numerous different cell lines can be used in the MN assay, there is (again) no expectation of success that any given cell line will lead to an effective screening process for a particular virus variant (or mutation or strain). Compounding to the unpredictability is the sheer number of different combinations in which the numerous starting dilutions, numerous cell lines, and numerous virus titers can be combined, with each combination producing different outcomes. Additional exacerbating factors include different incubation times prior to fixation, which also affect effectiveness.

FIGS. 1 through 12 provide specific workable ranges for starting materials in a screening process for different SARS-CoV-2 variants (e.g., the Washington isolate, Alpha variant, Gamma P.1 variant, Beta variant, Iota variant, Washington isolate, Delta variant, and Omicron variants, etc.). Although the general ELISA process is well known by those having skill in the art, FIGS. 1 through 12 demonstrate that specific parameters associated with different process steps within ELISA for specific SARS-CoV-2 variants are neither well known nor predictable.

Further expressions of the inventive concepts are set out in the following numbered clauses.
1. A process for performing microneutralization (MN) assays to quantify neutralizing antibodies to viruses, the process comprising:
   coating a plate with a monolayer of cells, the monolayer of cells being selected from the group consisting of:
      a monolayer of Vero E6 cells (BEI Resources Catalog Number NR-596); and
      a monolayer of Vero E6 cells modified to express higher levels of transmembrane protease, serine 2 (TMPRSS2) and angiotensin-converting enzyme 2 (ACE2) (BEI Resources Catalog Number NR-54970);
   inoculating the coated plate with a serum-included inoculum, the serum-included inoculum comprising:
      an immune serum; and
      a viral-infective dose of a variant of Severe Acute Respiratory Syndrome Coronavirus-2 (SARS-CoV-2), the immune serum diluted in approximately equal volumes with the viral-infective dose of the variant of SARS-CoV-2 and incubated for neutralization for approximately one hour (~1hr) at approximately thirty-seven degrees Celsius (~37°C) and at approximately five percent carbon dioxide (~5% CO2), the viral-infective dose of the variant of SARS-CoV-2 being one selected from the group consisting of:
         2,020 fifty-percent-tissue-culture-infective-dose-assays-per-milliliter (TCID₅₀/mL) input concentration of Alpha variant of SARS-CoV-2;
         90 TCID50/mL input concentration of Gamma P.1 variant of SARS-CoV-2;
         2,000 TCID50/mL input concentration of Beta variant of SARS-CoV-2;
         1,000 TCID50/mL input concentration of Iota variant of SARS-CoV-2;
         1,000 TCID50/mL input concentration of Washington isolate of SARS-CoV-2;
         1,000 TCID50/mL input concentration of Delta variant of SARS-CoV-2;
         5,000 TCID50/mL input concentration of Omicron BA.1 variant of SARS-CoV-2:
         30,000 TCID50/mL input concentration of Omicron BA.5 variant of SARS-CoV-2;
         10,000 TCID50/mL input concentration of Omicron XBB.1.5 variant of SARS-CoV-2;
      incubating the plate for a total incubating period at a temperature of ~37±2°C and a carbon dioxide (CO2) content of ~5±2%CO2, the total incubation period being one selected from the group consisting of:
         between twenty hours (20hrs) and twenty-eight hours (28hrs);
         between forty hours (40hrs) and forty-six hours (46hrs); and
         between sixty-eight hours (68hrs) and seventy-six hours (76hrs);
      removing the inoculation medium after the total incubation period;
      washing the plate with Hanks Buffered Salt Solution (HBSS);
      fixing the washed plate with approximately eighty percent (~80%) acetone;
      adding a primary antibody to the fixed plate, the primary antibody being a purified mouse monoclonal anti-coronavirus (COVID-19, MERS, SARS-CoV) nucleoprotein antibody;
      incubating the plate at a temperature of ~37±2°C for ~60±5 minutes (min) after adding the primary antibody;
      washing the plate having the primary antibody;
      adding a secondary antibody to the plate;
      incubating the plate at a temperature of ~37±2°C for ~60±5min after adding the secondary antibody, the secondary antibody being a goat anti-mouse immunoglobin G (IgG) antibody conjugated with horseradish peroxidase;
      washing the plate having the secondary antibody;
      applying 2,2'-azino-bis(3-ethylbenzothiazoline-6-sulfonic acid) (ABTS) solution to the plate;
      waiting for a period of ~304+5min;
      applying a stop solution to the plate; and
      reading the optical density of the plate at 405 nanometers (nm) with a 490nm reference filter.
2. The process of clause 1, wherein the monolayer of cells is the monolayer of Vero E6 cells.
3. The process of clause 1 or clause 2, wherein the viral-infective dose of the variant of SARS-CoV-2 is the 2,020 TCID50/mL input concentration of the Alpha variant of SARS-CoV-2.
4. The process of clause 1 or clause 2, wherein the viral-infective dose of the variant of SARS-CoV-2 is the 90 TCID50/mL input concentration of Gamma P.1 variant of SARS-CoV-2.
5. The process of clause 1 or clause 2, wherein the viral-infective dose of the variant of SARS-Cov-2 is the 2,000 TCID50/mL input concentration of Beta variant of SARS-CoV-2.
6. The process of clause 1 or clause 2, wherein the viral-infective dose of the variant of SARS-CoV-2 is the 1,000 TCID50/mL input concentration of Iota variant of SARS-CoV-2.
7. The process of clause 1 or clause 2, wherein the viral-infective dose of the variant of SARS-CoV-2 is the 1,000 TCID50/mL input concentration of Washington isolate of SARS-CoV-2.
8. The process of clause 1 or clause 2, wherein the viral-infective dose of the variant of SARS-CoV-2 is the 1,000 TCID50/mL input concentration of Delta variant of SARS-CoV-2.
9. The process of clause 1 or clause 2, wherein the viral-infective dose of the variant of SARS-CoV-2 is the 5,000 TCID50/mL input concentration of Omicron BA.1 variant of SARS-CoV-2.
10. The process of clause 1 or clause 2, wherein the viral-infective dose of the variant of SARS-CoV-2 is the 30,000 TCID50/mL input concentration of Omicron BA.5 variant of SARS-CoV-2.
11. The process of clause 1 or clause 2, wherein the viral-infective dose of the variant of SARS-CoV-2 is the 10,000 TCID50/mL input concentration of Omicron XBB.1.5 variant of SARS-CoV-2.
12. The process of any of clauses 1 to 11, wherein the total incubation period is between 40hrs-46hrs.
13. The process of any of clauses 1 to 11, wherein the total incubation period is between 68hrs-76hrs.
14. The process of any of clauses 1 to 11, wherein the total incubation period is between 20hrs-28hrs.
15. The process of clause 1, wherein the monolayer of cells is the monolayer of Vero E6-TMPRSS2/ACE2.
16. The process of clause 15, wherein the viral-infective dose of the variant of SARS-CoV-2 is the 1,000 TCID50/mL input concentration of Washington isolate of SARS-CoV-2.
17. The process of clause 15, wherein the viral-infective dose of the variant of SARS-CoV-2 is the 1,000 TCID50/mL input concentration of Delta variant of SARS-CoV-2.
18. The process of clause 15, wherein the viral-infective dose of the variant of SARS-CoV-2 is the 5,000 TCID50/mL input concentration of Omicron BA.1 variant of SARS-CoV-2.
19. The process of clause 15, wherein the viral-infective dose of the variant of SARS-CoV-2 is the 30,000 TCID50/mL input concentration of Omicron BA.5 variant of SARS-CoV-2.
20. The process of clause 15, wherein the viral-infective dose of the variant of SARS-CoV-2 is the 10,000 TCID50/mL input concentration of Omicron XBB.1.5 variant of SARS-CoV-2.
21. The process of clause 15, wherein the viral-infective dose of the variant of SARS-CoV-2 is the 2,020 TCID50/mL input concentration of Alpha variant of SARS-CoV-2.
22. The process of clause 15, wherein the viral-infective dose of the variant of SARS-CoV-2 is the 90 TCID50/mL input concentration of Gamma P.1 variant of SARS-CoV-2.
23. The process of clause 15, wherein the viral-infective dose of the variant of SARS-CoV-2 is the 2,000 TCID50/mL input concentration of Beta variant of SARS-CoV-2.
24. The process of clause 15, wherein the viral-infective dose of the variant of SARS-CoV-2 is the 1,000 TCID50/mL input concentration of Iota variant of SARS-CoV-2.
25. The process of any of clauses 15 to 24, wherein the total incubation period is between 20hrs-28hrs.
26. The process of any of clauses 15 to 24, wherein the total incubation period is between 40hrs-46hrs.
27. The process of any of clauses 15 to 24, wherein the total incubation period is between 68hrs-76hrs.
28. A process for performing microneutralization (MN) assays to quantify neutralizing antibodies to viruses, the process comprising:
   coating a plate with a monolayer of cells, the monolayer of cells being selected from the group consisting of:
      a monolayer of Vero E6 cells (BEI Resources Catalog Number NR-596); and
      a monolayer of Vero E6 cells modified to express higher levels of transmembrane protease, serine 2 (TMPRSS2) and angiotensin-converting enzyme 2 (ACE2) (BEI Resources Catalog Number NR-54970);
   inoculating the coated plate with a serum-included inoculum, the serum-included inoculum comprising:
      an immune serum; and
      a viral-infective dose of a variant of Severe Acute Respiratory Syndrome Coronavirus-2 (SARS-CoV-2), the immune serum diluted in equal volumes with the viral-infective dose of the variant of SARS-CoV-2 and incubated for neutralization for approximately one hour (~1hr) at approximately thirty-seven degrees Celsius (~37°C) and at approximately five percent carbon dioxide (~5% CO2), the viral-infective dose of the variant of SARS-CoV-2 being one selected from the group consisting of:
         2,020 fifty-percent-tissue-culture-infective-dose-assays-per-milliliter (TCID₅₀/mL) Alpha variant of SARS-CoV-2;
         90 TCID50/mL Gamma P.1 variant of SARS-CoV-2;
         2,000 TCID50/mL Beta variant of SARS-CoV-2;
         1,000 TCID50/mL Iota variant of SARS-CoV-2;
         1,000 TCID50/mL Washington isolate of SARS-CoV-2;
         1,000 TCID50/mL Delta variant of SARS-CoV-2;
         5,000 TCID50/mL Omicron BA.1 variant of SARS-CoV-2;
         30,000 TCID50/mL Omicron BA.5 variant of SARS-CoV-2;
         10,000 TCID50/mL Omicron XBB. 1.5 variant of SARS-CoV-2;
      incubating the plate for a total incubating period, the total incubation period being one selected from the group consisting of:
         between twenty hours (20hrs) and twenty-eight hours (28hrs);
         between forty hours (40hrs) and forty-six hours (46hrs); and
         between sixty-eight hours (68hrs) and seventy-six hours (76hrs);
      removing the inoculation medium after the total incubation period;
      washing the plate with Hanks Buffered Salt Solution (HBSS);
      fixing the washed plate with a fixative;
      adding a primary antibody to the fixed plate;
      incubating the plate after adding the primary antibody;
      washing the plate having the primary antibody;
      adding a secondary antibody to the plate;
      incubating the plate after adding the secondary antibody;
      washing the plate having the secondary antibody;
      applying 2,2'-azino-bis(3-ethylbenzothiazoline-6-sulfonic acid) (ABTS) solution to the plate;
      applying a stop solution to the plate; and
      reading the optical density of the plate.
29. The process of clause 28, wherein the monolayer of cells is the monolayer of Vero E6 cells.
30. The process of clause 28, wherein the monolayer of cells is the monolayer of Vero E6-TMPRSS2/ACE2.
31. The process of any of clauses 28 to 30, wherein the viral-infective dose of the variant of SARS-CoV-2 is the 2,020 TCID50/mL input concentration of the Alpha variant of SARS-CoV-2.
32. The process of any of clauses 28 to 30, wherein the viral-infective dose of the variant of SARS-CoV-2 is the 90 TCID50/mL input concentration of Gamma P.1 variant of SARS-CoV-2.
33. The process of any of clauses 28 to 30, wherein the viral-infective dose of the variant of SARS-CoV-2 is the 2,000 TCID50/mL input concentration of Beta variant of SARS-CoV-2.
34. The process of any of clauses 28 to 30, wherein the viral-infective dose of the variant of SARS-CoV-2 is the 1,000 TCID50/mL input concentration of Iota variant of SARS-CoV-2.
35. The process of any of clauses 28 to 30, wherein the viral-infective dose of the variant of SARS-CoV-2 is the 1,000 TCID50/mL input concentration of Washington isolate of SARS-CoV-2.
36. The process of any of clauses 28 to 30, wherein the viral-infective dose of the variant of SARS-CoV-2 is the 1,000 TCID50/mL input concentration of Delta variant of SARS-CoV-2.
37. The process of any of clauses 28 to 30, wherein the viral-infective dose of the variant of SARS-CoV-2 is the 5,000 TCID50/mL input concentration of Omicron BA.1 variant of SARS-CoV-2.
38. The process of any of clauses 28 to 30, wherein the viral-infective dose of the variant of SARS-CoV-2 is the 30,000 TCID50/mL input concentration of Omicron BA.5 variant of SARS-CoV-2.
39. The process of any of clauses 28 to 30, wherein the viral-infective dose of the variant of SARS-CoV-2 is the 10,000 TCID50/mL input concentration of Omicron XBB.1.5 variant of SARS-CoV-2.
40. The process of any of clauses 28 to 39, wherein the total incubation period is between 20hrs-28hrs.
41. The process of any of clauses 28 to 39, wherein the total incubation period is between 40hrs-46hrs.
42. The process of any of clauses 28 to 39, wherein the total incubation period is between 68hrs-76hrs.
43. The process of any of clauses 28 to 42, wherein the primary antibody is a purified mouse monoclonal anti-coronavirus nucleoprotein antibody.
44. The process of any of clauses 28 to 43, wherein the secondary antibody is a goat anti-mouse immunoglobin G (IgG) antibody conjugated with horseradish peroxidase.

Although exemplary embodiments have been shown and described, it will be clear to those of ordinary skill in the art that a number of changes, modifications, or alterations to the disclosure as described may be made. All such changes, modifications, and alterations should therefore be seen as within the scope of the disclosure.

## Claims

1. A process for performing microneutralization (MN) assays to quantify neutralizing antibodies to viruses, the process comprising:
coating a plate with a monolayer of cells, the monolayer of cells being selected from the group consisting of:
a monolayer of Vero E6 cells (BEI Resources Catalog Number NR-596); and
a monolayer of Vero E6 cells modified to express higher levels of transmembrane protease, serine 2 (TMPRSS2) and angiotensin-converting enzyme 2 (ACE2) (BEI Resources Catalog Number NR-54970);
inoculating the coated plate with a serum-included inoculum, the serum-included inoculum comprising:
an immune serum; and
a viral-infective dose of a variant of Severe Acute Respiratory Syndrome Coronavirus-2 (SARS-CoV-2), the immune serum diluted in approximately equal volumes with the viral-infective dose of the variant of SARS-CoV-2 and incubated for neutralization for approximately one hour (~1hr) at approximately thirty-seven degrees Celsius (~37°C) and at approximately five percent carbon dioxide (~5% CO₂), the viral-infective dose of the variant of SARS-CoV-2 being one selected from the group consisting of:
2,020 fifty-percent-tissue-culture-infective-dose-assays-per-milliliter (TCID₅₀/mL) input concentration of Alpha variant of SARS-CoV-2;
90 TCID₅₀/mL input concentration of Gamma P.1 variant of SARS-CoV-2;
2,000 TCID₅₀/mL input concentration of Beta variant of SARS-CoV-2;
1,000 TCID₅₀/mL input concentration of Iota variant of SARS-CoV-2;
1,000 TCID₅₀/mL input concentration of Washington isolate of SARS-CoV-2;
1,000 TCID₅₀/mL input concentration of Delta variant of SARS-CoV-2;
5,000 TCID₅₀/mL input concentration of Omicron BA.1 variant of SARS-CoV-2:
30,000 TCID₅₀/mL input concentration of Omicron BA.5 variant of SARS-CoV-2;
10,000 TCID₅₀/mL input concentration of Omicron XBB.1.5 variant of SARS-CoV-2;
incubating the plate for a total incubating period at a temperature of ~37±2°C and a carbon dioxide (CO₂) content of ~5±2%CO₂, the total incubation period being one selected from the group consisting of:
between twenty hours (20hrs) and twenty-eight hours (28hrs);
between forty hours (40hrs) and forty-six hours (46hrs); and
between sixty-eight hours (68hrs) and seventy-six hours (76hrs);
removing the inoculation medium after the total incubation period;
washing the plate with Hanks Buffered Salt Solution (HBSS);
fixing the washed plate with approximately eighty percent (~80%) acetone;
adding a primary antibody to the fixed plate, the primary antibody being a purified mouse monoclonal anti-coronavirus (COVID-19, MERS, SARS-CoV) nucleoprotein antibody;
incubating the plate at a temperature of ~37±2°C for ~60±5 minutes (min) after adding the primary antibody;
washing the plate having the primary antibody;
adding a secondary antibody to the plate;
incubating the plate at a temperature of ~37±2°C for ~60±5min after adding the secondary antibody, the secondary antibody being a goat anti-mouse immunoglobin G (IgG) antibody conjugated with horseradish peroxidase;
washing the plate having the secondary antibody;
applying 2,2'-azino-bis(3-ethylbenzothiazoline-6-sulfonic acid) (ABTS) solution to the plate;
waiting for a period of ~30±5min;
applying a stop solution to the plate; and
reading the optical density of the plate at 405 nanometers (nm) with a 490nm reference filter.

2. The process of claim 1, wherein:
the monolayer of cells is the monolayer of Vero E6 cells;
the viral-infective dose of the variant of SARS-CoV-2 is the 2,020 TCID₅₀/mL input concentration of the Alpha variant of SARS-CoV-2; and
the total incubation period is 40hrs-46hrs.

3. The process of claim 1, wherein:
the monolayer of cells is the monolayer of Vero E6 cells;
the viral-infective dose of the variant of SARS-CoV-2 is the 90 TCID₅₀/mL input concentration of Gamma P.1 variant of SARS-CoV-2; and
the total incubation period is 40hrs-46hrs.

4. The process of claim 1, wherein:
the monolayer of cells is the monolayer of Vero E6 cells;
the viral-infective dose of the variant of SARS-COV-2 is the 90 TCID₅₀/mL input concentration of Gamma P.1 variant of SARS-CoV-2; and
the total incubation period is 68hrs-76hrs.

5. The process of claim 1, wherein:
the monolayer of cells is the monolayer of Vero E6 cells;
the viral-infective dose of the variant of SARS-Cov-2 is the 2,000 TCID₅₀/mL input concentration of Beta variant of SARS-CoV-2; and
the total incubation period is 40hrs-46hrs.

6. The process of claim 1, wherein:
the monolayer of cells is the monolayer of Vero E6 cells;
the viral-infective dose of the variant of SARS-CoV-2 is the 1,000 TCID₅₀/mL input concentration of Iota variant of SARS-CoV-2; and
the total incubation period is 40hrs-46hrs.

7. The process of claim 1, wherein:
the monolayer of cells is the monolayer of Vero E6-TMPRSS2/ACE2;
the viral-infective dose of the variant of SARS-CoV-2 is the 1,000 TCID₅₀/mL input concentration of Washington isolate of SARS-CoV-2; and
the total incubation period is 40hrs-46hrs.

8. The process of claim 1, wherein:
the monolayer of cells is the monolayer of Vero E6-TMPRSS2/ACE2;
the viral-infective dose of the variant of SARS-CoV-2 is the 1,000 TCID₅₀/mL input concentration of Delta variant of SARS-CoV-2; and
the total incubation period is 40hrs-46hrs.

9. The process of claim 1, wherein:
the monolayer of cells is the monolayer of Vero E6-TMPRSS2/ACE2;
the viral-infective dose of the variant of SARS-CoV-2 is the 5,000 TCID₅₀/mL input concentration of Omicron BA.1 variant of SARS-CoV-2;
the total incubation period is 20hrs-28hrs.

10. The process of claim 1, wherein:
the monolayer of cells is the monolayer of Vero E6-TMPRSS2/ACE2;
the viral-infective dose of the variant of SARS-CoV-2 is the 5,000 TCID₅₀/mL input concentration of Omicron BA.1 variant of SARS-CoV-2;
the total incubation period is 40hrs-46hrs.

11. The process of claim 1, wherein:
the monolayer of cells is the monolayer of Vero E6-TMPRSS2/ACE2;
the viral-infective dose of the variant of SARS-CoV-2 is the 5,000 TCID₅₀/mL input concentration of Omicron BA.1 variant of SARS-CoV-2;
the total incubation period is 68hrs-76hrs.

12. The process of claim 1, wherein:
the monolayer of cells is the monolayer of Vero E6-TMPRSS2/ACE2;
the viral-infective dose of the variant of SARS-CoV-2 is the 30,000 TCID₅₀/mL input concentration of Omicron BA.5 variant of SARS-CoV-2;
the total incubation period is 20hrs-28hrs.

13. The process of claim 1, wherein:
the monolayer of cells is the monolayer of Vero E6-TMPRSS2/ACE2;
the viral-infective dose of the variant of SARS-CoV-2 is the 10,000 TCID₅₀/mL input concentration of Omicron XBB.1.5 variant of SARS-CoV-2;
the total incubation period is 20hrs-28hrs.

14. A process for performing microneutralization (MN) assays to quantify neutralizing antibodies to viruses, the process comprising:
coating a plate with a monolayer of cells, the monolayer of cells being selected from the group consisting of:
a monolayer of Vero E6 cells (BEI Resources Catalog Number NR-596); and
a monolayer of Vero E6 cells modified to express higher levels of transmembrane protease, serine 2 (TMPRSS2) and angiotensin-converting enzyme 2 (ACE2) (BEI Resources Catalog Number NR-54970);
inoculating the coated plate with a serum-included inoculum, the serum-included inoculum comprising:
an immune serum; and
a viral-infective dose of a variant of Severe Acute Respiratory Syndrome Coronavirus-2 (SARS-CoV-2), the immune serum diluted in equal volumes with the viral-infective dose of the variant of SARS-CoV-2 and incubated for neutralization for approximately one hour (~1hr) at approximately thirty-seven degrees Celsius (~37°C) and at approximately five percent carbon dioxide (~5% CO₂), the viral-infective dose of the variant of SARS-CoV-2 being one selected from the group consisting of:
2,020 fifty-percent-tissue-culture-infective-dose-assays-per-milliliter (TCID₅₀/mL) Alpha variant of SARS-CoV-2;
90 TCID₅₀/mL Gamma P.1 variant of SARS-CoV-2;
2,000 TCID₅₀/mL Beta variant of SARS-CoV-2;
1,000 TCID₅₀/mL Iota variant of SARS-CoV-2;
1,000 TCID₅₀/mL Washington isolate of SARS-CoV-2;
1,000 TCID₅₀/mL Delta variant of SARS-CoV-2;
5,000 TCID₅₀/mL Omicron BA.1 variant of SARS-CoV-2;
30,000 TCID₅₀/mL Omicron BA.5 variant of SARS-CoV-2;
10,000 TCID₅₀/mL Omicron XBB.1.5 variant of SARS-CoV-2;
incubating the plate for a total incubating period, the total incubation period being one selected from the group consisting of:
between twenty hours (20hrs) and twenty-eight hours (28hrs);
between forty hours (40hrs) and forty-six hours (46hrs); and
between sixty-eight hours (68hrs) and seventy-six hours (76hrs);
removing the inoculation medium after the total incubation period;
washing the plate with Hanks Buffered Salt Solution (HBSS);
fixing the washed plate with a fixative;
adding a primary antibody to the fixed plate;
incubating the plate after adding the primary antibody;
washing the plate having the primary antibody;
adding a secondary antibody to the plate;
incubating the plate after adding the secondary antibody;
washing the plate having the secondary antibody;
applying 2,2'-azino-bis(3-ethylbenzothiazoline-6-sulfonic acid) (ABTS) solution to the plate;
applying a stop solution to the plate; and
reading the optical density of the plate.

15. The process of claim 14, wherein:
the primary antibody is a purified mouse monoclonal anti-coronavirus nucleoprotein antibody; and
the secondary antibody is a goat anti-mouse immunoglobin G (IgG) antibody conjugated with horseradish peroxidase.
